# EUROPEAN PATENT APPLICATION

(11) **EP 0 963 972 A2**
(43) Date of publication of application: **15.12.1999**
(21) Application number: 99111112.1
(22) Date of filing: 08.06.1999
(51) Int. Cl.: C07C 29/143, C07B 53/00, C07C 269/06, C07C 271/16, C07C 319/20, C07C 323/41

(54) **Stereoselective reduction of carbonyl compounds**

(30) Priority: 09.06.1998 US 88618 P
(71) Applicant: F. HOFFMANN-LA ROCHE AG, 4070 Basel (CH)
(72) Inventor: Brown, Jack D., Westminster, Colorado 80020 (US); Cain, Robert O., Boulder, Colorado 80303 (US); Kopach, Michael E., Longmont, Colorado 80501 (US)
(74) Representative: Mezger, Wolfgang, Dr.

(57) **Abstract**

A process for the stereoselective reduction via a Meerwein-Ponndorf-Verley (MPV) reaction, of a carbonyl compound to the corresponding alcohol. A preferred starting carbonyl compound is a ketone compound wherein the carbonyl carbon is prochiral. The starting carbonyl compound is contacted with an MPV mediator such as aluminum isopropoxide. The reaction is conducted under mild conditions, e.g. temperatures of about 50°C or less, for less than four hours, and is capable of producing an excess (about 90% to about 97% or more) of a desired optically active chiral alcohol. The mild reaction conditions preserve the optical orientation of other asymmetric centers in the carbonyl compound starting material.

## Description

An important class of reactions for reducing aldehydes and ketones to the corresponding hydroxy compounds are the Meerwein-Ponndorf-Verley (MPV) reactions which proceed in most cases via a cyclic six-membered transition state in which both reductant and oxidant are coordinated to the metal center of a metal alkoxide catalyst.

Traditionally, aluminum compounds have not been used in catalytic (less than stoichiometric) amounts as mediators of the reduction reactions because ligand exchange is usually too slow. Instead, stoichiometric amounts of aluminum catalyst have been required (De Graauw, C.F., et al. (1994), "Meerwein-Ponndorf-Verley Reductions and Oppenauer Oxidations: An Integrated Approach," Synthesis (October 1, 1994) **10**:1007-1016).

Also, traditionally, an alcohol has been used as a hydrogen donor in the reactions:

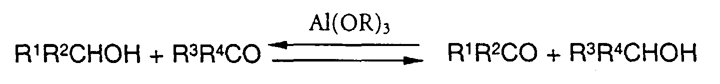

Diisopropoxyaluminum chloride has been partially substituted for aluminum isopropoxide as a mediator to lead to acceleration of rates of reaction (De Graauw et al. [1994] *supra*). However, stoichiometric amounts of the aluminum compound must be used.

To speed the reaction, the use of lanthanide, samarium, zirconium and hafnium complexes in lesser amounts as MPV mediators instead of aluminum compounds has been reported (De Graauw, C.F., et al. [1994] *supra*).

Rare earth trifluoromethanesulfonates have also been used as MPV mediators instead of aluminum oxides for such reactions. (Castellani, C.B., et al. (1993), "Rare earth trifluoromethanesulphonates as catalysts in some Meerwein-Ponndorf-Verley type reductions," J. Molec. Catalysis **85**:65-74.) This article reports that reaction rates increase as the cation size decreases and with the use of poorly coordinating counterions. Scandium trifluoromethanesulfonate was the best catalyst reported; however, the cost of this reagent precludes its application on a commercial scale.

In the oxidation of cyclohexanol to cyclohexanone coupled with reduction of the benzaldehyde to benzyl alcohol, it has been reported that trifluoroacetic acid (TFA) and other protic acids (hydrochloric acid, propionic acid and fluorosulfonic acid) increased the rate of the reactions at a less than stoichiometric molar ratio (vis-a-vis 0.5 equivalent). However, synthetic applications of this method are limited because TFA-aluminum oxide mixtures are potent catalysts for aldol condensation (Kow, R., et al. [1977], "Rate Enhancement of the Meerwein-Ponndorf-Verley-Oppenauer Reaction in the Presence of Proton Acids," J. Org. Chem. **42**:826-828).

The addition of trifluoroacetic acid (TFA) in stoichiometric amounts was reported to allow certain aluminum isopropoxide-mediated MPV reduction reactions to proceed at room temperature in about fifteen minutes. (Akamanchi, K.G. and Varalakshmy, N.R. [1995], "Aluminum Isopropoxide - TFA, a Modified Catalyst for Highly Accelerated Meerwein-Ponndorf-Verley (MPV) Reduction," Tetrahedron Letters **36**:3571-3572).

The same authors also reported that the use of one mole equivalent of isopropyl alcohol allowed molar ratios of aluminum isopropoxide to be reduced to as low as .0833 and of TFA to be reduced to as low as .0032 in the MPV reduction of p-nitrobenzaldehyde, a highly-reactive aldehyde, to the corresponding alcohol at room temperature. (Akamanchi, K.G. and Varalakshmy, R.N. [1995], "Truly Catalytic Meerwein-Ponndorf-Verley [MPV] Reduction," Tetrahedron Letters, **36**:5085-5088). The same article reports data showing that ketones require longer reaction times, e.g., six hours for cyclohexanone and 22-24 hours for other ketones.

A further article by Akamanchi, et al. reported the use of diisopropoxyaluminium trifluoroacetate, formed by reacting aluminum isopropoxide with TFA, as an MPV mediator to convert various aldehydes and ketones to alcohols in time periods ranging from fifteen minutes to twenty-four hours. Reduction of aldehydes required one equivalent of the reagent while ketones required 3-5 molar equivalents of the MPV mediator (Akamanchi, K.G., et al. [1997], "Diisopropoxyaluminium Trifluoroacetate: A New off the Shelf Metal Alkoxide Type Reducing Agent for Reduction of Aldehydes and Ketones," Synlett: 371-372).

None of the foregoing reports discuss the use of particular catalysts for stereoselective MPV reactions.

A chiral samarium (III) complex has been used at ambient temperatures as an MPV mediator in 2-propanol for the stereoselective MPV reduction of aryl alkyl ketones. However, it was determined that the enantioselectivity of the reduction is highly sensitive to the size of the alkyl substituent. For example, increasing the size of the alkyl group from methyl to ethyl resulted in an enantioselectivity reduction from 96% enantiomeric excess (e.e.) to 73% e.e. (Evans, D.A., et al. (1993), "A Chiral Samarium-Based Catalyst for the Asymmetric Meerwein-Ponndorf-Verley Reduction," J. Am. Chem. Soc. **115**:9800-9801).

Auxiliary compounds have been used to promote stereoselective reduction reactions. Asymmetric MPV reductions of acetophenone mediated by isopropanol in the presence of the chiral diols (4)-(-)-1-phenyl-2,2-dimethylpropane-1,3-diol and (4)-(+)-1,1'-binaphthalene-2,2'-diol using aluminum triisopropoxide have been reported. (Xianming, H. and Kellogg, R.M. [1996], "Asymmetric reduction and Meerwein-Ponndorf-Verley reaction of prochiral aromatic ketones in the presence of optically pure 1-aryl-2,2-dimethylpropane-1,3-diols," Recl. Trav. Chim. Pays-Bas. **115**:410-417). However, these reactions requiring complex auxiliary compounds are expensive due to the high cost of the chiral auxiliary compounds.

A chiral alcohol with a thiol moiety has been used to convert α,β-unsaturated ketones to the corresponding saturated secondary alcohols using dimethylaluminum chloride. While the reduction provided the alcohols in high diastereoselectivity, the subsequent reductive desulfurization step consumes a stoichiometric equivalent of chiral mercapto alcohol (Nishide, K., et al. [1996], "Asymmetric 1,7-Hydride Shift: The Highly Asymmetric Reduction of α,β-Unsaturated Ketones to Secondary Alcohols via a Novel Tandem Michael Addition - Meerwein-Ponndorf-Verley Reduction," J. Am. Chem. Soc. **118**:13103-13104).

A zeolite MPV mediator has been used to convert 4-tert-butylcyclohexanone to the cis-isomer of 4-tert-butylcyclohexanol with greater than 95 percent stereoselectivity. (Creyghton, E.J., et al [1997], "Stereoselective Meerwein-Ponndorf-Verley and Oppenauer reactions catalysed by zeolite BEA," J. Molec. Catalysis A: Chemical **115**: 457-472.)

U.S. Patents 5,523,463 and 5,591,885 report the use of stoichiometric or greater amounts of aluminum isopropoxide for reduction of methyl (S)-(1-benzyl-3-chloro-2-oxo-propyl)-carbamate to the corresponding alcohol in an isopropanol solution. The product, (1S,2S)-(1-benzyl-3-chloro-2-hydroxy-propyl)-carbamate was obtained as an 89% pure stereoisomer.

MPV reactions have been used to reduce methyl [3-chloro-2-oxo-(R)-1-(phenylthiomethyl)-propyl]-carbamate to the corresponding alcohol using stoichiometric or greater amounts of aluminum isopropoxide in isopropanol to achieve 75% yield of isomerically pure methyl (1R,2S)-[3-chloro-2-hydroxy-1-(phenylthiomethyl)-propyl] - carbamate after recrystallization. It has been suggested that this procedure would be effective with lower alkyl analogs and analogs with other halide substituents, and that stereoisomers of up to 90% purity might be obtained.

None of the foregoing references disclose or suggest MPV reactions using aluminum isopropoxides in catalytic, i.e. less than stoichiometric, amounts to provide stereoselectivity at the prochiral ketone from the reduction reaction. In addition, none of these references teach or suggest stereoselective MPV reactions for aryl and thioaryl carbamate starting materials in the absence of isopropanol or other alcoholic solvents or reactants. Further, none of these references disclose such reactions for the preservation of adjacent chiral centers while creating new chiral centers having desired orientations.

Stereoselective and stereospecific reactions for producing desired optically active alcohols are needed for the preparation of intermediate and final pharmaceutical products. The use of aluminum alkoxides in stereoselective and stereospecific MPV reactions is desirable. Where feasible, it is even more desirable from a cost and environmental standpoint to reduce the amount of aluminum compound required, and therefore the amount of used reagent requiring disposal. Further, it is desired both from a cost standpoint and to achieve optimal stereoselectivity or stereospecificity to conduct the reaction at temperatures below about 50°C, and preferably below about 45°C, in economically feasible periods of time, e.g. within one to three hours, at yields greater than about 85% and with a ratio of desired to undesired optically isomer of 95:5 or more.

### DEFINITIONS

The following definitions and abbreviations are used herein:
C* means a chiral carbon atom
"Ac" means acetyl.
"Boc" means t-butyloxycarbonyl.
"CBZ" means benzyloxycarbonyl.
"MPV" means Meerwein-Ponndorf-Verley.
"MSA" means methane sulfonic acid
"TFA" means trifluoro acetic acid

The term "stereoselective" in reference to the process hereof means that an excess of a desired optically active product is produced over the undesired optically active product. Preferably this excess is more than about 80%, i.e. a ratio of desired optically active product to undesired optically active product of more than about 90:10.

The term "highly stereoselective" in reference to the process hereof means a process producing an excess of a desired optically active produce of about 90% or more. Most preferably, this excess is at least about 94-96%. Stereoselectivities may be optimized through selection of MPV and other reagents most suitable for the specific carbonyl-containing compound being reduced.

A "carbonyl compound" is any compound containing a carbonyl group (-C=O). In the processes of this invention, upon reduction of the C=O group at C-2 as described above to a C^{*}-OH, the carbon atom becomes a "chiral carbon atom," i.e., the carbon is bonded to four different substituents, so that two diastereomeric alcohols may be produced, with a desired diastereomer being produced in excess. Where there is an existing chiral carbon atom in the carbonyl compound, the reduction will potentially result in a mixture of diastereomeric isomers. The term "enantiomeric excess" as used herein refers to an excess of one enantiomer of a d, l mixture. Preferably, where there is a pre-existing chiral carbon, e.g., C-1 as described above, the stereochemical integrity of this carbon atom is preserved in the formation of the alcohol.

The term "diastereomeric excess" refers to an excess of a compound having the desired newly-formed chirality and the preserved pre-existing chiral carbon over the undesired diastereomeric compound. The term "enantiomeric excess" includes diastereomeric excess and is used to describe situations involving compounds having one or more chiral carbons, the enantiomeric excess referring specifically to the newly-formed chirality.

"Reduction" of the carbonyl compound to the corresponding alcohol refers to conversion of the -C=O group to a -C*-OH group.

The "corresponding alcohol" to the "carbonyl compound" herein is one in which such reduction of the -C=O group to a -C*-OH group has occurred.

"Stoichiometric amounts" as used with respect to MPV mediators of this invention means molar equivalents based on moles of starting carbonyl compound.

"Contacting" reaction components with each other refers to providing a medium and/or reaction chamber in which the reaction components are placed together so that they can react with each other. Preferably, the reaction components are suspended or dissolved in a carrier fluid which is a liquid medium such as toluene, benzene, methanol, ethanol, isopropanol, butanol, tetrahydrofuran, and other suitable carriers known to the art, from which products can be easily purified, e.g., by distillation, after the reaction is as complete as desired.

"A time sufficient to produce an excess of a desired optically active product" means a time sufficient to form the desired optically active product in detectable amounts. In stereoselective and highly stereoselective processes, the desired optically active product may be formed in detectable amounts before detectable amounts of the undesired optically active product are formed. Preferably, the amount of time the reactants are maintained in contact is at least sufficient to obtain about an 85% yield (based on both optically active products) and preferably about a 90% to 95% yield based on both optically active products. Preferably the time required for the reaction is less than about four hours, more preferably two hours or less, and most preferably about one hour or less.

The term "amine group" refers to a primary, secondary, or tertiary amine group.

A "3-halo-1-amino-2-propanone" is one in which there is an amine group (-NH₂, -NHR or -NR₂) attached to the carbon at C-1. Most of the 3-halo-1-amino-2-propanones of this invention will be substituted with hydrocarbyl groups at C-1 and C-2.

An amido ethyl ketone has an amide group (R-CO-NR'-) where R is hydrocarbyl and R' is H or R) attached to the carbon next to the carbonyl group of the ketone starting material. Again, most of these ketones will be substituted at C-1 and C-2.

A "chiral carbon,' as is known to the art, is one having four different substituents attached thereto. Molecules containing chiral carbons may exist as optically active isomers containing both (S) and (R) forms.

A "prochiral carbon," as is known to the art, is one bonded to three dissimilar moieties and capable of becoming chiral upon reaction to replace one of its four bonds, i.e., with respect to the ketone compounds described herein, the prochiral carbon of the carbonyl is bonded to O and two dissimilar moieties so that upon reduction of the carbonyl to the corresponding alcohol the carbonyl carbon becomes chiral, i.e., bonded to four dissimilar moieties.

The term "hydrocarbyl' is used herein to refer generally to organic radicals comprised of carbon chains to which hydrogen and optionally other elements are attached. CH₂ or CH groups and C atoms of the carbon chains of the hydrocarbyl may be replaced with one or more heteroatoms (i.e., non-carbon atoms). Suitable heteroatoms include but are not limited to O, S and N atoms. The term hydrocarbyl includes, but is not limited to alkyl, alkenyl, alkynyl, ether, thioether, aminoalkyl, hydroxylalkyl, thioalkyl, aryl and heterocyclic aryl groups, groups which have a mixture of saturated and unsaturated bonds, carbocyclic rings and combinations of such groups. The term also includes straight-chain, branched-chain and cyclic structures or combinations thereof. Hydrocarbyl groups are optionally substituted. Hydrocarbyl substitution includes substitution at one or more carbons in the group by moieties containing heteroatoms. Suitable substituents for hydrocarbyl groups include but are not limited to OH, SH, NH₂, COH, CO₂H, ORₐ, SRₐ, NRₐR_{b}, CONRₐR_{b}, and halogens where Rₐ and R_{b} independently are alkyl, unsaturated alkyl or aryl groups.

The term "alkyl" takes its usual meaning in the art and is intended to include straight-chain, branched and cycloalkyl groups. The term includes, but is not limited to, methyl, ethyl, n-propyl, isopropyl, n-butyl, sec-butyl, isobutyl, tert-butyl, n-pentyl, neopentyl, 2-methylbutyl, 1-methylbutyl, 1-ethylpropyl, 1,1-dimethylpropyl, n-hexyl, 1-methylpentyl, 2-methylpentyl, 3-methylpentyl, 4-methylpentyl, 3,3-dimethylbutyl, 2,2-dimethylbutyl, 1,1-dimethylbutyl, 2-ethylbutyl, 1-ethylbutyl, 1,3-dimethylbutyl, n-heptyl, 5-methylhexyl, 4-methylhexyl, 3-methylhexyl, 2-methylhexyl, 1-methylhexyl, 3-ethylpentyl, 2-ethylpentyl, 1-ethylpentyl, 4,4-dimethylpentyl, 3,3-dimethylpentyl, 2,2-dimethylpentyl, 1,1-dimethylpentyl, n-octyl, 6-methylheptyl, 5-methylheptyl, 4-methylheptyl, 3-methylheptyl, 2-methylheptyl, 1-methylheptyl, 1-ethylhexyl, 1-propylpentyl, 3-ethylhexyl, 5,5-dimethylhexyl, 4,4-dimethylhexyl, 2,2-diethylbutyl, 3,3-diethylbutyl, and 1-methyl-1-propylbutyl. Alkyl groups are optionally substituted. Lower alkyl groups are C₁-C₆ alkyl and include among others methyl, ethyl, n-propyl, and isopropyl groups.

The term "cycloalkyl" refers to alkyl groups having a hydrocarbon ring, particularly to those having rings of 3 to 7 carbon atoms. Cycloalkyl groups include those with alkyl group substitution on the ring. Cycloalkyl groups can include straight-chain and branched-chain portions. Cycloalkyl groups include but are not limited to cyclopropyl, cyclobutyl, cyclopentyl, cyclohexyl, cycloheptyl, cyclooctyl, and cyclononyl. Cycloalkyl groups can optionally be substituted.

The term "unsaturated alkyl" group is used herein generally to include alkyl groups in which one or more carbon-carbon single bonds have been converted to carbon-carbon double or triple bonds. The term includes alkenyl and alkynyl groups in their most general sense. The term is intended to include groups having more than one double or triple bond, or combinations of double and triple bonds. Unsaturated alkyl groups include, without limitation, unsaturated straight-chain, branched or cycloalkyl groups. Unsaturated alkyl groups include without limitation: vinyl, alkyl, propenyl, isopropenyl, butenyl, pentenyl, hexenyl, hexadienyl, heptenyl, cyclopropenyl, cyclobutenyl, cyclopentenyl, cyclopentadienyl, cyclohexenyl, cyclohexadienyl, 1-propenyl, 2-butenyl, 2-methyl-2-butenyl, ethynyl, propargyl, 3-methyl-1-pentynyl, and 2-heptynyl. Unsaturated alkyl groups can optionally be substituted.

Substitution of alkyl, cycloalkyl and unsaturated alkyl groups includes substitution at one or more carbons in the group by moieties containing heteroatoms. Suitable substituents for these groups include but are not limited to OH, SH, NH₂, COR, CO₂H, OR_{c}, SR_{c}, NR_{c}R_{d}, CONR_{c}R_{d}, and halogens, particularly chlorines and bromines where R_{c} and R_{d}, independently, are alkyl, unsaturated alkyl or aryl groups. Preferred alkyl and unsaturated alkyl groups are lower alkyl, alkenyl or alkynyl groups having from 1 to about 3 carbon atoms.

The term "aryl" is used herein generally to refer to aromatic groups which have at least one ring having a conjugated pi electron system and includes without limitation carbocyclic aryl, aralkyl, heterocyclic aryl, biaryl groups and heterocyclic biaryl, all of which can be optionally substituted. Preferred aryl groups have one or two aromatic rings.

Compounds possessing substantially the same properties as the unsubstituted aryl compounds of this invention and which can be prepared in the same manner and are equivalents thereof are those wherein the aryl group bears one, two or more simple substituents including, but not limited to, lower alkyl, e.g., methyl, ethyl, butyl; halo, e.g., chloro, bromo; nitro; sulfato; sulfonyloxy; carboxy; carbo-lower-alkoxy, e.g., carbomethoxy, carbethoxy; amino; mono- and di-lower-alkylamino, e.g., methylamino, ethylamino, dimethylamino, methylethylamino; amido; hydroxy; lower-alkoxy, e.g., methoxy, ethoxy; and lower-alkanoyloxy, e.g., acetoxy.

"Carbocyclic aryl" refers to aryl groups in which the aromatic ring atoms are all carbons and includes without limitation phenyl, biphenyl and napthalene groups.

"Aralkyl" refers to an alkyl group substituted with an aryl group. Suitable aralkyl groups include among others benzyl, phenethyl and picolyl, and may be optionally substituted. Aralkyl groups include those with heterocyclic and carbocyclic aromatic moieties.

"Heterocyclic aryl groups" refers to groups having at least one heterocyclic aromatic ring with from 1 to 3 heteroatoms in the ring, the remainder being carbon atoms. Suitable heteroatoms include without limitation oxygen, sulfur, and nitrogen. Heterocyclic aryl groups include among others furanyl, thienyl, pyridyl, pyrrolyl, N-alkyl pyrrolo, pyrimidyl, pyrazinyl, imidazolyl, benzofuranyl, quinolinyl, and indolyl, all optionally substituted.

"Heterocyclic biaryl" refers to heterocyclic aryls in which a phenyl group is substituted by a heterocyclic aryl group ortho, meta or para to the point of attachment of the phenyl ring to the decalin or cyclohexane. Heterocyclic biaryl includes among others groups which have a phenyl group substituted with a heterocyclic aromatic ring. The aromatic rings in the heterocyclic biaryl group can be optionally substituted.

"Biaryl" refers to carbocyclic aryl groups in which a phenyl group is substituted by a carbocyclic aryl group ortho, meta or para to the point of attachment of the phenyl ring to the decalin or cyclohexane. Biaryl groups include among others a first phenyl group substituted with a second phenyl ring ortho, meta or para to the point of attachment of the first phenyl ring to the decalin or cyclohexane structure. Para substitution is preferred. The aromatic rings in the biaryl group can be optionally substituted.

Aryl group substitution includes substitutions of H atoms by non-aryl groups at one or more carbon atoms or where possible at one or more heteroatoms in aromatic rings. Suitable substituents for aryl groups include among others, alkyl groups, unsaturated alkyl groups, halogens, OH, SH, NH₂, COH, CO₂H, ORₑ, SRₑ, NRₑR_{f}, CONRₑR_{f}, where Rₑ and R_{f} independently are alkyl, unsaturated alkyl or aryl groups. Preferred substituents are OH, SH, ORₑ, and SRₑ where Rₑ is a lower alkyl, i.e. an alkyl group having from 1 to about 3 carbon atoms. Other preferred substituents are halogens, more preferably chlorine or bromine, and lower alkyl and unsaturated lower alkyl groups having from 1 to about 3 carbon atoms. Substituents include bridging groups between aromatic rings in the aryl group, such as -CO₂-, -CO-, -O-, -S-, -NH-, -CH=CH- and -(CH₂)ₗ- where l is an integer from 1 to about 5, and particularly -CH₂-. Examples of aryl groups having bridging substituents include phenylbenzoate. Substituents also include moieties, such as -(CR₂)ₗ-, -O-(CH₂)ₗ- or -OCO-(CH₂)ₗ-, where l is an integer from about 2 to 7, as appropriate for the moiety, which bridge two ring atoms in a single aromatic ring as, for example, in a 1, 2, 3, 4-tetrahydronaphthalene group. Alkyl and unsaturated alkyl substituents of aryl groups can in turn optionally be substituted as described *supra* for substituted alkyl and unsaturated alkyl groups.

The terms "alkoxy group" and "thioalkoxy group" (also known as mercaptide groups, the sulfur analog of alkoxy groups) take their generally accepted meaning. Alkoxy groups include but are not limited to methoxy, ethoxy, n-propoxy, isopropoxy, n-butoxy, sec-butoxy, isobutoxy, tert-butoxy, n-pentyloxy, neopentyloxy, 2-methylbutoxy, 1-methylbutoxy, 1-ethyl propoxy, 1,1 -dimethylpropoxy, n-hexyloxy, 1-methylpentyloxy, 2-methylpentyloxy, 3-methylpentyloxy, 4-methylpentyloxy, 3,3-dimethylbutoxy, 2,2-dimethoxybutoxy, 1-1-dimethylbutoxy, 2-ethylbutoxy, 1-ethylbutoxy, 1,3-dimethylbutoxy, n-pentyloxy, 5-methylhexyloxy, 4-methylhexyloxy, 3-methylhexyloxy, 2-methylhexyloxy, 1-methylhexyloxy, 3-ethylpentyloxy, 2-ethylpentyloxy, 1-ethylpentyloxy, 4,4-dimethylpentyloxy, 3,3-dimethylpentyloxy, 2,2-dimethylpentyloxy, 1,1-dimethylpentyloxy, n-octyloxy, 6-methylheptyloxy, 5-methylheptyloxy, 4-methylheptyloxy, 3-methylheptyloxy, 2-methylheptyloxy, 1-methylheptyloxy, 1-ethylhexyloxy, 1-propylpentyloxy, 3-ethylhexyloxy, 5,5-dimethylhexyloxy, 4,4-dimethylhexyloxy, 2,2-diethylbutoxy, 3,3-diethylbutoxy, 1-methyl-1-propylbutoxy, ethoxymethyl, n-propoxymethyl, isopropoxymethyl, sec-butoxymethyl, isobutoxymethyl, (1-ethyl propoxy)methyl, (2-ethylbutoxy)methyl, (1-ethylbutoxy)methyl, (2-ethylpentyloxy)methyl, (3-ethylpentyloxy)methyl, 2-methoxyethyl, 1-methoxyethyl, 2-ethoxyethyl, 3-methoxypropyl, 2-methoxypropyl, 1-methoxypropyl, 2-ethoxypropyl, 3-(n-propoxy)propyl, 4-methoxybutyl, 2-methoxybutyl, 4-ethoxybutyl, 2-ethoxybutyl, 5-ethoxypentyl, and 6-ethoxyhexyl. Thioalkoxy groups include but are not limited to the sulfur analogs of the alkoxy groups specifically listed *supra*.

"Optional" or "optionally" means that the subsequently described event or circumstance may or may not occur, and that the description includes instances where said event or circumstance occurs and instances in which it does not. For example, "optionally substituted phenyl" means that the phenyl radical may or may not be substituted and that the description includes both unsubstituted phenyl radicals and phenyl radicals wherein there is substitution.

"α-Amino acids'' as used herein include naturally occurring and commercially available amino acids and racamates as well as optical isomers thereof. Typical natural and commercially available amino acids are glycine, alanine, serine, homoserine, threonine, valine, norvaline, leucine, isoleucine, norleucine, aspartic acid, glutamic acid, lysine, ornithine, histidine, arginine, cysteine, homocysteine, methionine, phenylalanine, homophenylalanine, phenylglycine, o-, m-, and p-tyrosine, tryptophan, glutamine, asparagine, proline and hydroxyproline. An α-amino acid residue is a residue-NHCH(R)C(O)-, wherein R is an α-amino acid side chain, except for the amino acid residues of proline and hydroxyproline which are -N(CH₂-CH₂-CH₂)CHC(O)- and -N(CH-CHOHCH₂)CHC(O)-, respectively. An α-amino acid side chain is a residue found on the α-carbon of an α-amino acid as defined herein, where the residue is ,e.g., hydrogen (side chain of glycine), methyl (side chain of alanine), or is a radical bonded to the α-carbon by a methylene (-CH₂-), or phenyl group.

"Optionally protected α-amino acid residue" means an α-amino acid residue having an optionally protected α-amino acid side chain.

"Optionally protected α-amino acid side chain" includes an unprotected α-amino acid side chain such as the H in glycine, the CH₃ in alanine, and the CH₂OH in serine; or if the side chain includes heteroatoms such as oxygen, sulfur, or nitrogen, the heteroatoms on the side chain can optionally be protected with an O-, S-, or N-protecting group.

An "O-, S-, or N-protecting group" is a radical attached to an oxygen, sulfur, or nitrogen atom, respectively, which radical serves to protect the oxygen, sulfur, or nitrogen functionality against undesired reactions and/or to modify the properties of the molecule to which it is attached (e.g., its solubility, lipophilicity, bioavailability, etc.). Such protecting groups are well known in the art, and many are described in "The Peptides," E. Gross and J. Meienhofer, Eds., Vol. 3, Academic Press, NY (1981), and "Chemistry of the Amino Acids," J. P. Greenstein and M. Winitz, Vol. 2, J. Wiley and Sons, NY (1961).

"N-Protecting groups" for amino functionalities of amino acids, at the peptide N-terminal, or on amino acid side chains are well known in the art. An exemplification of known amino N-protecting groups is included in "The Peptides," E. Gross and J. Meienhofer, Eds., Academic Press, NY (1981), Vol. 3, Chapter 1; "Protective Groups in Organic Synthesis," T. W. Greene, J. Wiley and Sons, NY (1981), Chapter 7; and "Chemistry of the Amino Acids," J. P. Greenstein and M. Winitz, J. Wiley and Sons, NY (1961), Vol. 2, pp. 885-924; other (less well-known) N-protecting groups include the methoxysuccinyl group (CH3-OCOCH₂-CH₂-CO-), the hydroxysuccinyl group (HOOCCH₂-CH₂-CO-), the p-methoxycarbonyl-benzoyl group (p-CH₃-OC₆H₄-CO-), the p-phenylsulfonamidocarbonyl-benzoyl group (p-C₆H₅-SO₂-NHCO-C₆H₄-CO-), and the 2-(1-adamantyl)-ethoxycarbonyl group.

Generally, these N-protecting groups can be considered to fall within five classes: N-acyl, N-alkoxycarbonyl, N-arylmethoxycarbonyl, N-arylmethyl, and N-arylsulfonyl protecting groups. An N-acyl protecting group is a lower alkyl carbonyl residue, a trifluoroacetyl residue, a methoxysuccinyl residue (CH₃ -OCOCH₂ -CH₂-CO-), a hydroxysuccinyl residue (HOOCCH₂ -CH₂-CO-) or a phenylcarbonyl(benzoyl) residue optionally substituted on the phenyl ring with p-methoxycarbonyl, p-phenylsulfonamidocarbonyl (p-C₆H₅-SO₂NHCO-), p-methoxy, or p-nitro. An N-alkoxycarbonyl protecting group is a lower alkoxycarbonyl residue or a 2-(1-adamantyl)ethoxycarbonyl residue. An N-arylmethoxycarbonyl protecting group is a 9-fluorenemethoxycarbonyl residue (Fmoc); or benzyloxycarbonyl residue which can optionally be substituted on the aromatic ring with p-methoxy, p-nitro, p-chloro, or o-(N,N-dimethyl-carboxamido). An N-arylmethyl protecting group is a benzyl residue, which can optionally be substituted on the aromatic ring with p-methoxy, p-nitro, or p-chloro. An N-arylsulfonyl protecting group is a phenylsulfonyl residue, which can optionally be substituted on the aromatic ring with p-methyl ("tosyl") or p-methoxy.

"N-protecting groups" for guanidino functionalities on arginine amino acid side chains are known in the art, and described in "The Peptides," Vol. 3, pp. 60-70 and "Chemistry of the Amino Acids," Vol. 2, pp. 1068-1077, as cited earlier. These include the nitro, p-toluenesulfonyl, p-methoxyphenylsulfonyl, CBZ, and Boc N-protecting groups.

"N-protecting groups" for imidazole functionalities on histidine amino acid side chains are known in the art, and described in "The Peptides," Vol. 3, pp. 70-80, and "Chemistry of the Amino Acids," Vol. 2, pp.1060-1068, as cited earlier. These include the benzyl, triphenylmethyl (trityl), 2,4-dinitrophenyl, p-toluenesulfonyl, benzoyl, and CBZ N-protecting groups.

"N-protecting groups" for indole functionalities on tryptophan amino acid side chains are known in the art and described in "The Peptides," Vol. 3, pp. 82-84, as cited earlier. These include the formyl and CBZ N-protecting groups.

"O-protecting groups" for hydroxy functionalities on amino acid side chains are known in the art and described in "The Peptides," Vol. 3, pp.169-201, and "Chemistry of the Amino Acids," Vol. 2, pp. 1050-1056, as cited earlier. For aliphatic hydroxy functionalities, suitable O-protecting groups include benzyl, tert-butyl, and methyl groups. For aromatic hydroxy functionalities, suitable O-protecting groups include the benzyl, tert-butyl, methyl, CBZ, and tosyl groups.

"O-protecting groups" for carboxy functionalities on amino acid side chains are well known in the art and described in "The Peptides," Vol. 3, pp. 101-135, as cited earlier, and include the methyl, ethyl, tert-butyl, and benzyl groups.

"S-protecting groups" for thiol functionalities on amino acid side chains are known in the art, and described in "The Peptides," Vol. 3, pp. 137-167, and "Chemistry of the Amino Acids," pp. 1077-1092, as cited earlier. These include the methyl, tert-butyl, benzyl, p-methoxyphenylmethyl, ethylamino-carbonyl, and CBZ groups.

This invention provides a process for the stereoselective reduction of a series of carbonyl compounds to the corresponding optically active alcohols. The method is particularly useful for producing intermediates in the preparation of protease inhibitors such as those useful in the treatment of HIV, e.g., saquinavir and nelfinavir.

The method comprises contacting a compound comprising a carbonyl group having chiral center and a prochiral carbon atom with a Meerwein-Ponndorf-Verley (MPV) mediator at a temperature of about 50°C or less, for a sufficient time to produce an excess of the desired optically active alcohol, normally less than four hours.

In one embodiment, particularly useful in the preparation of a saquinavir intermediate the method comprises:
(a) providing a carbonyl compound comprising a carbonyl moiety and a chiral carbon atom,
(b) contacting said carbonyl compound with a hydrogen donor, such as isopropyl alcohol, and a Meerwein-Ponndorf-Verley (MPV) mediator, such as aluminum isopropoxide, in equal to or preferably less than a stoichiometric amount, i.e., equal to or preferably less than one molar equivalent based on the amount of starting carbonyl compound;
(c) maintaining said carbonyl compound, MPV mediator and hydrogen donor in contact at a temperature of about 50°C or less, preferably about 42°C or less, for a time, preferably about four hours or less, and more preferably about two hours or less, sufficient to produce an excess of the desired optically active alcohol, preferably an enantiomeric excess of at least about 80%, i.e. a ratio of about 90:10.

In another embodiment, particularly useful for preparation of a nelfinavir intermediate, the method comprises:
(1) providing a carbonyl compound comprising a carbonyl group and a chiral carbon atom;
(2) contacting said carbonyl compound with an MPV mediator in stoichiometric or greater amounts, preferably in the presence of a non-polar solvent, in the absence of alcoholic reactants; and
(3) maintaining said carbonyl compound and MPV mediator in contact at a temperature of about 50°C or less, preferably about 42°C or less, more preferably about 25°C or less and most preferably about 10°C or less, for a time, preferably about four hours or less, and more preferably about two hours or less, sufficient to produce an excess of the desired optically active alcohol, preferably an enantiomeric excess of at least about 80%, i.e. a ratio of about 90:10, preferably about 97:3.

This embodiment is especially useful for carbonyl compounds which are sensitive to decomposition, as the absence of alcoholic solvents or reactants helps prevent such decomposition processes.

The desired optically active alcohol may be purified from the reaction components, including any undesired enantiomers and/or diastereomers, by chromatographic separation, crystallization, or other methods known in the art.

In MPV reactions, the hydrogen donor is oxidized while the carbonyl compound is reduced. Typically the equilibrium of the reaction is shifted in favor of reduction of the carbonyl compound to the corresponding alcohol by removing the oxidation product of the hydrogen donor (in a preferred embodiment in which isopropyl alcohol is the hydrogen donor, acetone may be removed, e.g., by distillation); however, in the present process, good yields have been obtained without the necessity for removing the oxidation product of the hydrogen donor.

The carbonyl compound may be any carbonyl-containing compound not having structures which would interfere with the desired MPV reaction. Structures which might interfere with this reaction include other carbonyl groups, or unprotected sulfur or nitrogen groups which could compete with the hydrogen donor for hydrogen. If such possibly competing functional groups are present, the functional groups may be protected by any of the methods conventional in the art (as is more specifically described below), including the use of protecting groups, during the MPV reaction and, optionally, these protecting groups may be removed thereafter if desired.

Preferably the starting material is a ketone, and more preferably the starting material is a 3-halo-1-amino-2-propanone core having an amino substituent on the first carbon atom (C-1) of the propyl group, and having a carbonyl functional group at the second carbon atom (C-2) of the propyl group. Preferably, a bulky hydrophobic group (i.e., one or more aromatic rings which are optionally substituted as described below) via an intervening lower alkyl group, is also attached to the first carbon atom (C-1) of the propyl group. The halogen may be directly attached to C-3 or may be linked thereto via an intervening lower alkyl group to the carbonyl group. Other substituents may also be present at C-1 or C-3 so long as they do not interfere with the MPV reaction as discussed above.

The hydrogen donor as referred to herein is any compound capable of supplying a hydrogen to the reduction reaction, e.g., an alcohol, especially any easily oxidizable primary or secondary alcohol. Preferred hydrogen donors are C₁₋₆ alcohols, such as ethanol, isopropanol, isoborneol, butan-2-ol or 2-methylbutan-1-ol. A most preferred hydrogen donor is isopropanol. Preferably the hydrogen donor is present in greater than stoichiometric amounts, e.g., preferably about five molar equivalents based on the starting ketone.

The Meerwein-Ponndorf-Verley (MPV) mediator is any mediator known to the art capable of mediating the reaction. Such mediators include aluminum compounds such as aluminum isopropoxides, aluminum tert-butoxide, aluminum phenoxide, aluminum sec-butoxide, and others known to the art as such mediators, acid salts of such aluminum compounds, e.g., diisopropoxy aluminum methane sulfonate, diisopropoxy aluminum trifluoroacetate, etc. Other metal alkoxides capable of mediating the reduction reaction include the lanthanide alkoxides, e.g., samarium alkoxides, gadolinium alkoxides, as well as zirconium and hafnium complexes. Preferably the MPV mediator used herein is an aluminum isopropoxide. The term "aluminum isopropoxide" includes aluminum di- and tri-isopropoxides, the corresponding aluminum chloro-di-isopropoxides, and acid salts thereof, preferably methanesulfonates thereof. Similarly, other aluminum compounds described herein include the "di-" and "tri-" forms. In a preferred embodiment the MPV mediator is used in amounts of about 0.5 equivalents based on the amount of starting carbonyl compound and isopropanol is used as a hydrogen donor in amounts of at least about double stoichiometric. For less stable carbonyl starting materials, such as those having arylthio substituents on a carbon adjacent to the prochiral carbonyl carbon and/or other unstable aryl groups, it is preferred that the process be conducted in the absence of alcoholic reactants or solvents such as isopropyl alcohol, and using stoichiometric or greater amounts of MPV mediator.

The MPV reduction process using a hydrogen donor agent and less than stoichiometric amounts of MPV mediator is preferably conducted in the presence of a strong acid. The strong acid may be any strong acid known in the art. A strong acid is defined herein as those with Pkₐ values that are less than 2. Methanesulfonic acid, trifluoroacetic acid (TFA), trifluoromethanesulfonic acid and perfluoroethylenesulfonic acid are preferred strong acids for use herein. More preferred are methanesulfonic acid, ethanesulfonic acid, or other C₁₋₅-alkyl sulfonic acid and most preferred is methanesulfonic acid. As a stronger acid than TFA, methanesulfonic acid may be used in lesser amounts than TFA, preferably less than stoichiometric amounts. The acid is used in an amount sufficient to ensure efficient conversion, preferably in an amount equal to about 0.5 equivalents based on the amount of carbonyl compound present. In some embodiments, the MPV mediator and the acid are pre-reacted to form a complex prior to contacting with the carbonyl compound starting material. In this case, the MPV mediator may be an acid salt or complex such as aluminum isopropoxide methanesulfonate.

The MPV reaction not using an alcoholic reactant or solvent is preferably conducted without strong acids and a nonpolar solvent such as toluene. Hexane is preferably not used in the reaction mixture, and acids are also preferably not used.

The MPV reduction processes of this invention are efficient, with yields generally greater than about 85%, and normally greater than about 95% based on amount of the selected optically active isomer as a percentage of the amount of the starting carbonyl compound. Most preferably 1% or less of the starting carbonyl compound remains unreacted.

In preferred embodiments, the carbon atom next to the carbonyl moiety of the carbonyl compound starting material, defined above as C-1, is a chiral carbon atom. Also in preferred embodiments, the compound is an alkyl halide, preferably a chloride, with the chlorine atom at C-3. The process is especially suited for preserving the orientation of the chiral carbon atom at C-1 in the starting material, as the reaction conditions are relatively mild.

In preferred embodiments, there is a protected amino group (-NHR or -NR₂) attached to the chiral carbon atom (C-1) of the starting material, i.e. the starting material is a 1-amino-2-propanone.

A further class of carbonyl compound starting materials are propanones selected from the group consisting of compounds of the formula: 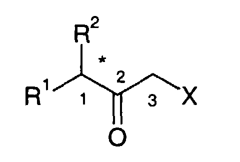 wherein * indicates a chiral carbon atom; R¹ and R² can be any substituents which do not adversely affect the reaction, and are, independently, hydrocarbyl groups; and X is Cl, F, Br or I, preferably Cl. R² is preferably a hydrophobic moiety, preferably alkyl, aryl, arylthio, aralkyl or arylthioalkyl.

A class of preferred carbonyl compound starting materials are ketones of the formula: 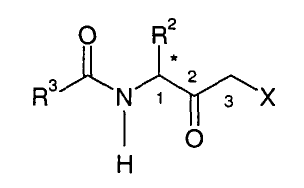 wherein *, R² and X are as set forth above and R³ is a hydrocarbyl group.

A further class of preferred carbonyl compound starting materials useful in this invention is the group consisting of compounds of the formula: 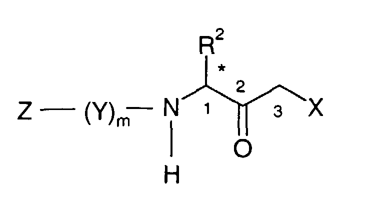 wherein * and X are as set forth above;
m is 0, 1 or 2;
Z is H, an N-protecting group, or a substituted alkanoyl or aroyl group;
Y is, independently, an optionally protected α-amino acid residue; and
R₂ is arylthio, arylthioalkyl or an optionally protected α-amino acid side chain.

Such side chain may be, e.g., H (from Glycin), methyl (from alanine), phenyl (from phenylglycin), benzyl (from phenyl alanine), isopropyl (from valine), 2-butyl (from isoleucine), methythioethyl (from methionine), hydroxymethyl (from serine) or hydroxyethyl (from threonine). The reactive side chains from amino acids with, e.g., hydroxy, amino or carboxy groups may be protected.

Further preferred classes of starting materials useful in this invention are compounds of formulae

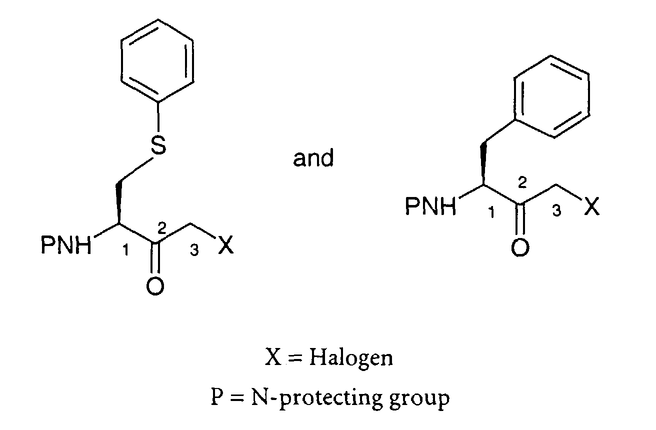

Most preferably, the carbonyl compound starting material is methyl [3-chloro-2-oxo-(S)-1-(benzyl)-propyl]-carbamate or benzyl [3-chloro-2-oxo-(R)-1-(phenylthiomethyl)-propyl] carbamate.

The production of a key intermediate of saquinavir requires a reduction of methyl [3-chloro-2-oxo-(S)-1-(benzyl)-propyl]-carbamate to produce (1S,2S)-methyl [3-chloro-2-hydroxy-1-(benzyl)-propyl]-carbamate. The process of this invention is highly stereoselective, producing an enantiomeric excess of the desired enantiomer over the undesired (1S,2R)-methyl [3-chloro-2-hydroxy-1-(benzyl)-propyl]-carbamate of at least 90%, i.e. a ratio of the (1S,2S) to (1S,2R) product of at least about 95:5, and preferably about 92 to 94% or higher, i.e. a ratio of (1S,2S) to (1S,2R) of at least about 96:4 to 97:3 or higher.

The production of a key intermediate of nelfinavir requires a reduction of benzyl [3-chloro-2-oxo-(R)-1-(phenylthiomethyl)-propyl]-carbamate to produce benzyl (1R,2S)-[3-chloro-2-hydroxy-1-(phenylthiomethyl)-propyl]-carbamate.

In the conversion of the saquinavir carbonyl compound intermediate, methyl [3-chloro-2-oxo-(S)-1-(benzyl)-propyl] -carbamate, to the corresponding alcohol, the starting carbonyl compound is contacted with the hydrogen donor, preferably by suspending an amount of starting carbonyl compound in a carrier fluid, preferably toluene at a solvent ratio of about 6:1 to about 8:1. The more concentrated the carbonyl compound is in the carrier fluid, the better the throughput.

To the formed slurry is added the MPV mediator, preferably aluminum sec-butoxide or aluminum isopropoxide in less than a stoichiometric amount, preferably at about 0.3 to about 0.7 molar equivalents, and most preferably at about 0.5 molar equivalents based on the starting ketone. Higher amounts of MPV mediator may be used; however, as this will result in an increase in the waste stream, it is desired to minimize the amount used. Adding substantially less MPV mediator, e.g. 0.1 or less equivalents, will result in significantly slower reaction rates.

A hydrogen donor is also added, preferably isopropanol in a greater than stoichiometric amount, preferably about 2 to about 7 equivalents and more preferably about 5 to about 6 equivalents based on the starting ketone. Higher amounts of hydrogen donor may be added if desired without adverse effects. Lower amounts may also be used, however, if less than about three equivalents are used, the reaction rates will be significantly reduced.

The reaction mixture is preferably cooled in order to minimize the mild exothermic reaction, preferably to about 0°C to about 15°C, and an acid, preferably methanesulfonic acid, is added to the reaction mixture. At least about 0.5 equivalents of acid based on the starting ketone is preferably used. More than 0.5 equivalents of acid up to an amount which causes decomposition of the reactants and products may be used and will increase the rate of reaction; however, due to reagent cost, it is preferred that 0.5 equivalents or less be used. Less than about 0.5 equivalents may also be used, down to an amount causing the reaction to slow to the point where yields as claimed herein cannot be achieved in less than three or four hours, all as may be easily determined by those skilled in the art without undue experimentation.

The reaction mixture is then preferably heated to above room temperature, more preferably between about 38°C and about 42°C, and preferably no more than about 50°C. Higher temperatures may result in the decomposition of the ketone substrate, the formation of impurities, and reduction of stereoselectivity. Too little heat slows down the reaction. For example, at about 30°C to about room temperature, the reaction works quite well giving superior optical purity, but takes about 3 to 6 hours longer than at the higher temperature.

The reactants are maintained in contact, preferably at the optimal temperature discussed above, until a desired amount of the selected optically active alcohol has been produced. The reaction mixture may be sampled at various points and analyzed, e.g. by HPLC, to determine the amount of starting material remaining. Typically, less than 1% starting material will remain after about one to two hours.

The reaction mixture is then cooled, e.g. to about 15±5°C to minimize the exothermic effect of the reaction. Acid is then added, e.g. aqueous hydrochloric acid, to bring the mixture to a pH of about 3 to dissociate the MPV mediator. This prevents further undesired reactions. The carrier fluid is then removed, e.g. by evaporation or vacuum distillation. Additional hydrogen donor may be added to assist in the removal of the carrier fluid, and this may be repeated until the residual level of carrier fluid is minimal, e.g. less than about 5% as measured by gas chromatography.

The reaction product may then be purified, for example by further acidification, to pH about 1, preferably with dilute hydrochloric acid or other suitable acid, and heating to about 50-55°C to dissolve the product, followed by distillation to crystallize the product. Crystallization may be aided by cooling and the product isolated, e.g. by filtration. Washing the product in the hydrogen donor and water and drying may also be done to complete the process.

To convert the nelfinavir carbonyl compound intermediate, benzyl [3-chloro-2-oxo-(R)-1-phenylthiomethyl)-propyl]-carbamate to the corresponding alcohol, the starting carbonyl compound is contacted with an MPV mediator, preferably aluminum sec-butoxide or aluminum tert-butoxide, at a molar ratio of preferably one or more, e.g., up to about 1.1, in toluene under nitrogen at about 5-10°C. Preferably tetrahydrofuran (THF) and water or alcohols are absent or kept to amounts less than about 0.5%, preferably less than 0.05% for water and alcohols. Hexane is also preferably kept out of the reaction mixture.

Alternatively, benzyl [3-chloro-2-oxo-(R)-1-phenylthiomethyl)-propyl]-carbamate may be converted to the corresponding alcohol by contacting the starting carbonyl compound with an MPV mediator, preferably aluminum isopropoxide, in a carrier fluid, preferably toluene. The reaction mixture is preferably cooled to about 10°C and an acid, preferably methanesulfonic acid, is added to the mixture. The reaction mixture is warmed to about 40°C for about 60 minutes and then cooled to about 25°C until less than 1% of the ketone remains. The reaction mixture is quenched and worked up as described above.

The reaction proceeds to completion (less than about 1% unreacted starting material) over a period of about two to three hours at 10°C. The reaction mixture may then be heated to about 65°C over a period of about an hour until homogeneity occurs, and the aqueous layer removed and organics distilled off. Hexane may then be added to form about a 40% hexane/toluene mixture, and the mixture cooled to crystallize product. The process achieves a 92% yield with a 97:3 ratio of desired to undesired diastereomer.

Repeatable yields of 90-92% of the desired optically active isomer based on the carbonyl compound starting material are achieved using the processes of this invention with a ratio of desired to undesired optically active product of about 96:5 to 97:3 or better.

The present invention is further illustrated by the following EXAMPLES.

### Example 1.

Starting material, the carbonyl compound, methyl [3-chloro-2-oxo-(S)-1-(benzyl)-propyl]-carbamate was suspended in toluene at a solvent ratio of 6:1 to 8:1. To the formed slurry was charged 0.5 equivalents of aluminum isopropoxide and 6-7 equivalents of isopropanol. The mixture was cooled to 10°C and methanesulfonic acid (0.5 equivalents) was charged, maintaining the pot temperature in the range of 10°C to 15°C. The slurry was then heated to 40± 2°C and held at that temperature for 30 minutes. The reaction mixture (homogenous at this stage) was analyzed for residual chloromethylketone. The reaction was allowed to proceed for a further 30 minutes at which stage the mixture was again sampled and analyzed. Starting carbonyl compound was present at less than 1% as measured by HPLC. Cooling was then applied to reduce the batch temperature to about 20-25°C.

The slurry was then acidified to about pH 3 with enough aqueous hydrochloric acid to dissociate the aluminum complex and the mixture was stripped to a thick paste to remove most of the toluene. The mixture was diluted with an equivalent amount of isopropanol equal to the toluene/water mixture removed by vacuum distillation and this again was stripped to a thick paste and diluted with an amount of isopropanol equal to that removed by vacuum distillation. At this stage the residual level of toluene in the slurry was less than 5% as measured by gas chromatography.

Acidification to pH 1 with dilute hydrochloric acid followed by distillation yielded (after dilution with water at 50-55°C) a crystalline slurry of the desired product. The slurry was cooled to 24-28°C and isolated by filtration. The wet cake was washed first with 20% isopropanol in water, then neat water, and dried at about 60°C under vacuum to constant weight.

The desired product, methyl (1S,2S)-[3-chloro-2-hydroxy-1-(benzyl)-propyl]-carbamate, was obtained in a number of repetitions in 87-90% yield based on carbonyl compound starting material. The crude stereoselectivity of the reduction was 94:6 to 95:5 (S,S to S,R).

### Example 2.

Methyl [3-chloro-2-oxo-(S)-1-(benzyl)-propyl]-carbamate in the amount of 5 mmol (1.27 g) was added to toluene (25 ml) to give a solvent ratio of 20:1. One equivalent of isopropyl alcohol was added along with 0.1 equivalents of aluminum isopropoxide. The mixture was cooled to 0-5°C and 0.08 equivalents of methanesulfonic acid was added. The slurry was allowed to warm to room temperature and stirred for 36-40 hours. Solubility of the chloroketone in toluene is poor. After sixteen hours the conversion to the corresponding alcohol was 24% by area normalized HPLC. The stereoselectivity of the reduction was 93:7 (S,S to S,R).

### Example 3.

Varying conditions of temperature, time, and solvent ratios were tested using the reactants of Examples 1 and 2. Results are shown in Table 1. The results showed that a temperature of 40°C is generally better than 30°C since while the lower temperature gives better optical purity, an excess amount of starting carbonyl compound (ketone) remains unreacted even after long reaction times. Solvent ratios of 6:1 work well; however, increased solvent ratios may be desirable to improve agitation and transfer between vessels.

**Table 1**

| Ketone (g) | Toluene (ml) | Temp (□C) | Solvent Ratio | Reaction time (hr) | e.e. (%) | Ketone remaining (%) |
|---|---|---|---|---|---|---|
| 3.835 | 30.6 | 40 | 8:1 | 1.0 | 92% | 0.9 |
| 3.825 | 23.0 | 30 | 6:1 | 6.0 | 94% | 2.3 |
| 21.20 | 127.0 | 40 | 6:1 | 1.5 | 93% | 0.93 |
| 42.4 | 424.0 | 40 | 10:1 | 1.0 | 92% | 0.87 |
| 31.8 | 189.0 | 40 | 6:1 | 1.0 | 93% | 0.72 |

### Example 4.

Available starting material for the reaction of the foregoing Examples may contain about 16-17% hexane and about 1-2% THF when diluted with isopropanol. Therefore, tests were done to determine the effects of these impurities on the reaction. Results are shown in Table 2. These impurities appear to have no adverse effect on the reaction.

**Table 2**

| Ketone (g) | Hexane (ml) | THF (ml) | Temp (□C) | Reaction time (hr) | e.e. (%) | Ketone remaining (%) |
|---|---|---|---|---|---|---|
| 3.835 | 6.22 | 0.37 | 40 | 1.5 | 93% | 0.61 |
| 3.825 | 6.22 | 0.00 | 50 | 1.0 | 93% | 0.88 |

### Example 5.

Scale-up experiments were run using 20-50 g of starting material. Results are shown in Table 3.

**Table 3**

| Ketone (g) | Yield (%) | (S,R) (%) | (S,S) (%) | Reduction Time (hr) | Crude e.e. (%) | Residual Starting Material (%) |
|---|---|---|---|---|---|---|
| 21.2 | 89.9 | 0.09 | 99.15 | 1.5 | 93% | 0.93 |
| 42.4 | 88.7 | 0.07 | 99.17 | 1.0 | 92% | 0.87 |
| 31.8 | 88.8 | 0.05 | 99.57 | 1.0 | 93% | 0.72 |

In all cases, yields were excellent and (S,R) levels were within normal ranges. The high level of (S,R) in the first experiment of Table 3 was due to overstripping of the batch resulting in a lower than normal ratio of isopropyl alcohol to water (about 22%). Normal isopropyl alcohol to water ratios are in the 30:70 range with about a 2% spread. Optical purities at the crude reaction stage were also much improved at 92-93% enantiomeric excess (E.E.) relative to 88-90% for the best prior art process. Levels of oxazolidinone and other impurities continued to decrease.

### Example 6.

The starting material and procedure of Example 1 were used, substituting 0.5 eq. of aluminum tri-n-butoxide, aluminum tri-sec-butoxide, or aluminum tri-t-butoxide for the aluminum isopropoxide catalyst used in Example 1. Yields of the S,S isomer at 30 minutes to four hours for each compound are compared with those for aluminum isopropoxide shown in Table 4.

**Table 4**

| S,S Isomer Yields Using 0.5 Equivalents of Various Catalysts | | | | | | | |
|---|---|---|---|---|---|---|---|
| Catalyst | Time [mm.] | | | | | | |
| | 0 | 30 | 60 | 90 | 120 | 180 | 240 |
| Aluminum tri-isopropoxide | 0 | 87.3 | 93.8 | 94.6 | 95.1 | 94.3 | 94.2 |
| Aluminum tri-butoxide | 0 | 11.57 | 19.66 | 26.2 | 31.78 | 41.23 | 46.9 |
| Aluminum sec-butoxide | 0 | 93.21 | 95.61 | 95.42 | 94.71 | 94.04 | 94.68 |
| Aluminum tert-butoxide | 0 | 88.8 | 93.92 | 94.51 | 94.34 | 94.02 | 93.5 |

### Example 7.

The starting material of Example 1 in the amount of 4.20 g (16.4 mmol), isopropanol in the amount of 7.0 ml (about 6-7 equivalents), and 2.0 g (0.5 eq., 8.2 mmol) aluminum tri-sec-butoxide dissolved in 40 ml toluene was charged to a three-necked round bottom 250 ml flask fitted with a condenser, inert gas system, heating pad, temperature control system and mechanical agitator. The mixture was cooled to 10-15□C and methanesulfonic acid in the amount of 0.5 ml (8.2 mmol, 0.5 eq.) was added. The mixture was warmed to 40□C over 30 minutes, at which point the reaction mixture was homogenous. The temperature was maintained at 40±2□C until less than 1% of the starting material remained (about 0.75 to 1.0 hours). HPLC analysis of the reaction mixture showed a reduction selectivity at the end of the reaction of 95.21% (S,S-isomer) vs. 3.01% (S,R-isomer) giving an enantiomeric excess of 94.8%. The oxazolidinone by-product was present at 0.86%.

### Example 8.

Under a nitrogen atmosphere, a three-neck jacketed 1 l apparatus was charged with 117.4 mM (28.9 g 1.05 eq) aluminum tri-sec-butoxide and 150 ml toluene (3.7 volumes), and the mixture was cooled to less than 5°C with stirring under nitrogen. Solid benzyl [3-chloro-2-oxo-(R)-1-phenylthiomethyl)-propyl]-carbamate (40.6 g) was slurried in 175 ml toluene (4.3 volumes) and transferred into the reaction vessel in a single portion (about 5 minutes addition time). The mixture was warmed to 10°C over 30 minutes and held at 10°C until reaction completion. The total volume of the reaction mixture was 440 mL (10.8 volumes). Less than 1% of the starting material remained unreacted at 2.0-3.0 hours total reaction time.

The mixture was then cooled to 5°C, and 70 ml 25% HCl was slowly added over 30 minutes (exotherm from 5-12°C to 65°C over one hour) with rigorous stirring until homogeneity occurred. The total volume occupied was 575 ml (14.2 volumes). The aqueous layer was removed at 65°C. The organic phase was then washed once with 75 mL of water at 60-65°C with vigorous stirring at 60-65°C for five minutes prior to separation. Three volumes of distillate (120 ml) were removed at reduced pressure (30 minute removal time) distillate head temperature 50-55°C at 150mm Hg, pot temperature 65°C). The mixture was held at 65-68°C and 3.7 volumes of hexane (150 ml) was added over one hour to form a 40% hexane/toluene mixture while maintaining a pot temperature of 65°C. The mixture was held at 65-66°C for one hour after completion of the hexane addition, then cooled from 65-66°C at a rate of 20 degrees per hour. The mixture was then held at 5°C for one hour (total crystallization time 4-6 hours). The mixture was filtered at 25°C and filtration assisted with four volumes of 1:1 toluene/hexane. The wetcake was washed with 4-5 volumes of hexane (total filtration time 15-30 minutes). The solid was dried under reduced pressure (150 mm Hg) with nitrogen bleed at 40°C for four hours.

By this method a 92% yield of benzyl (1R,2S)-[3-chloro-2-hydroxy-1-(phenylthiomethyl)-propyl] carbamate was obtained with extremely high purity (>98.8%). Selectivity for the desired diastereomer in the crude reduction mixture was greater than 97.0:3:0. Less than 1.5% of the chloroalcohol was lost in the mother liquor. The ratio of the desired to undesired diastereomer in the mother liquor was 2:5. The solvent volume was successfully reduced by 20% during the reduction, 60% for acid quench.

During the acid quench viscous suspension results which does not stir well. The suspension broke at 30°C while heating prior to phase separation. Precipitation of some solid chloroalcohol was observed toward the end of the distillation. However, vigorous agitation for 30 minutes at 67°C restored a homogeneous solution. Crystallization began to occur after about 20% hexane was added.

### Example 9

The procedure of Example 8 was repeated using different reducing agents to compare with the MPV reagent aluminum sec-butoxide. The results, set forth in Table 5, show the superiority of the MPV reagent in achieving highly stereoselective results -- (1R,2S):(1R,2R) = 97:3.

### Example 10

The procedure of Example 8 was repeated using a number of MPV reagents and a number of different solvents. The results, set forth in Table 6, show superior stereoselectivity when non-polar solvents are used.

### Example 11

The procedure of Example 8 was repeated using a number of different MPV reagents and a number of different acids. The results, set forth in Table 7, show high stereoselectivities as high as 98.0:2.0 when methylsulfonic acid and aluminum isopropoxide are premixed before adding to the starting material.

Upon reading the present specification, various alternative embodiments will become obvious to the skilled artisan. For example, the invention has been illustrated with reference to the stereoselective preparation of saquinavir and nelfinavir intermediates. As will be readily apparent to those skilled in the art, intermediates to a wide range of other protease inhibitors and other compounds may be stereoselectively prepared by analogous reactions using minor variations ascertainable without undue experimentation. These variations are to be considered within the scope and spirit of the present invention.

**Table 5**

| **Entry** | **Reagent** | **Solvent** | **Δ°C** | **R,S:R,R** | **Time** | **Conversion** |
|---|---|---|---|---|---|---|
| 1 | NaBH₄ | THF/MeOH | 25 | 60:40 | 0.1 h | >99% |
| 2 | BU₄NBH₄ | Toluene | -78 | 72:28 | 0.5 h | >99% |
| 3 | Me₄NBH₄ | Toluene | -78 | 67:33 | 0.5 h | >99% |
| 4 | Li-9-BBN hydride* | Toluene/THF | -78 | 71:29 | 0.1 h | >99% |
| 5 | ** | Toluene | -78 | 67:33 | 0.5 h | >99% |
| 6 | DIBAL-H*** | Toluene | -78 | 46:54 | 0.2 h | >99% |
| 7 | LiAlH₄ | Toluene | -78 | 50:50 | 1.0 h | 99% |
| 8 | Al(sec-BuO)₃ | Toluene | 10 | 97:3 | 2.0 h | >99% |

| | | | | | | |
|---|---|---|---|---|---|---|
| * Lithium 9-borabicyclo[3.3.1]nonane hydride | | | | | | |
| ** Sodium bis-(2-methoxyethoxy)-aluminum hydride | | | | | | |
| *** Diisobutylaluminum hydride | | | | | | |

**Table 6**

| **Entry** | **Reagent/Amt (eq.)** | **Solvent** | **Δ°C** | **R,S:R,R** | **Time** | **Conversion** |
|---|---|---|---|---|---|---|
| 1 | Al(IprO)₃, 1.00 | IPrOH | 60 | 90:10 | 3 h | >99% |
| 2 | Al(IprO)₃, 1.03 | Toluene | 25 | 92.7:7.3 | 24 h | 95% |
| 3 | Al(sec-BuO)₃, 1.04 | Toluene | 25 | 96.0:4.0 | 1 h | >99% |
| 4 | Al(sec-BuO)₃, 1.00 | Toluene | 10 | 97.0:3.0 | 2 h | >99% |
| 5 | Al(sec-BuO)₃, 1.04 | 1:1 Toluene/hexanes | 25 | 97.0:3.0 | 2.5 h | >99% |
| 6 | Al(sec-BuO)₃, 1.05 | THF | 25 | 86:14 | 4 h | >99% |
| 7 | Al(sec-BuO)₃, 1.00 | 2-butanol | 25 | 92.1:7.9 | 1 h | >99% |
| 8 | Al(sec-BuO)₃, 1.05 | CH₃CN | 25 | 92.0:8.0 | 1 h | >99% |
| 9 | Al(sec-BuO)₃, 1.03 | CH₂Cl₂ | 25 | 96.4:3.6 | 1 h | 99% |
| 10 | Al(sec-BuO)₃, 1.02 | MTBE | 25 | 93.5:6.5 | 2.5 h | >99% |
| 11 | Zr(IprO)₄IPA, 1.05 | Toluene | 25 | 86.5:13.5 | 24h | 53% |

| | | | | | | |
|---|---|---|---|---|---|---|
| MTBE = Methyl tert.-butyl ether | | | | | | |

**Table 7**

| **Entry** | **Reagent/ Amt (eq.)** | **Acid/ Amt (eq.)** | **Δ°C** | **R,S:R,R** | **Time** | **Conversion** |
|---|---|---|---|---|---|---|
| 1 | Al(IprO)₃, 1.03 | MSA/0.99 | 10-25 | 97.0:3.0 | 4 h | >99% |
| 2 | Al(IprO)₃, 1.10 | MSA/1.00 | 40 | 96.3:3.7 | <2 h | >99% |
| 3* | Al(IprO)₃, 1.40 | MSA/1.30 | 25 | 98.0:2.0 | 8 h | >99% |
| 4 | Al(IprO)₃, 1.02 | MSA/0.50 | 10-25 | 96.0:4.0 | 4 h | >99% |
| 5 | Al(IprO)₃, 1.00 | HOT**/1.00 | 25 | 85.0:15.0 | 5 h | 55% |
| 6 | Al(IprO)₃, 1.03 | TFA/1.01 | 25 | 77.0:23.0 | 12 h | >99% |
| 7 | Al(IprO)₃, 1.00 | TCA***/1.00 | 25 | 79.0:21.0 | 24 h | 90% |
| 8 | Al(sec-BuO)₃, 1.04 | MSA/1.00 | 10-25 | 96.8:3.2 | 2 h | >99% |
| 9 | Al(sec-BuO)₃, 1.03 | HOAc/0.99 | 25 | 94.0:6.0 | 17 h | 37% |
| 10 | Al(sec-BuO)₃, 1.05 | (+)Camp.****/1.04 | 25 | 84.0:16.0 | 17 h | 91% |
| 11 | Zr(IprO)₄IPA, 1.02 | MSA/1.00 | 25 | 95.1:4.9 | 24h | 99% |

| | | | | | | |
|---|---|---|---|---|---|---|
| * MSA and Al(IprO)₃ premixed before addition of ketone. | | | | | | |
| ** Tri-fluoro methane sulfonic acid. | | | | | | |
| *** Trichloroacetic acid. | | | | | | |
| **** Camphor sulfonic acid. | | | | | | |

## Claims

1. A process for the stereoselective reduction of a carbonyl compound to the corresponding optically active alcohol comprising contacting a compound comprising a chiral center and a carbonyl group having a prochiral carbon atom with a Meerwein-Ponndorf-Verley (MPV) mediator at a temperature of about 50°C or less, for a sufficient time to produce an excess of the desired optically active alcohol, normally less than four hours.

2. The process of claim 1 wherein said MPV mediator is present in less than stoichiometric amounts, and a hydrogen donor is added in at least about double stoichiometric amounts.

3. The process of claim 1 or claim 2 characterized in that it is conducted in the presence of a strong acid.

4. The process of claim 3 wherein said acid is selected from the group consisting of trifluoroacetic acid, p-toluenesulfonic acid, a C₁₋₅ alkyl sulfonic acid, such as methanesulfonic acid or trifluoromethanesulfonic acid.

5. The process of any one of claims 1 to 4 wherein said MPV mediator is selected from the group consisting of aluminum isopropoxide, aluminum tert-butoxide, aluminum phenoxide, and aluminum sec-butoxide.

6. The process of claim 5 wherein said MPV mediator is aluminum sec-butoxide or aluminum isopropoxide.

7. The process of any one of claims 1 to 6 wherein said hydrogen donor is an alcohol.

8. The process of claim 7 wherein said alcohol is isopropanol.

9. The process of claim 1 wherein said MPV mediator is present in stoichiometric amounts
or greater and said process is conducted in the absence of alcoholic solvents or reactants.

10. The process of any one of claims 1 to 9 wherein the carbonyl compound comprises a chiral carbon atom adjacent to the prochiral carbon atom of said carbonyl group.

11. The process of claim 10 wherein said carbonyl compound is selected from the group of compounds represented by formula: 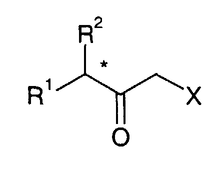 wherein
R' and R² are, independently, hydrocarbyl;
X is Cl, F, Br or I and
* indicates a chiral carbon atom.

12. The process of claim 11 wherein R¹ is hydrocarbyl-CO-NH-; and the asterisk, R² and X are as defined above.

13. The process of claim 11 or claim 12 wherein R² is alkyl, aryl, aralkyl, arylthio or arylthioalkyl.

14. The process of claim 11 wherein R¹ is
Z―(Y)ₘ―NH―
wherein
m is 0, 1 or 2;
Z is H or an N-protecting group;
Y is, independently, an optionally protected α-amino acid residue; and
R₂ is arylthio, arylthioalkyl or an optionally protected α-amino acid side chain.

15. The process of any one of claims 1-14 wherein the carbonyl compound is methyl [3-chloro-2-oxo-(S)-1-(benzyl)-propyl]-carbamate.

16. The process of any one of claims 1-14 wherein the carbonyl compound is benzyl [3-chloro-2-oxo-(R)-1-(phenylthiomethyl)-propyl] carbamate.
